# EUROPÄISCHE PATENTSCHRIFT

(11) **EP 2 865 274 B1**
(45) Veröffentlichungstag und Bekanntmachung des Hinweises auf die Patenterteilung: **04.03.2020**
(21) Anmeldenummer: 13190052.4
(22) Anmeldetag: 24.10.2013
(51) Int. Cl.: A23K 10/12, A23K 20/142, A23K 20/158, A23K 40/10, A23K 40/30

(54) **L-Aminosäure enthaltendes Futtermitteladditiv**
Food additive containing L-amino acid
Additifs de nourriture pour animaux contenant des acides animés L

(43) Veröffentlichungstag der Anmeldung: 29.04.2015
(73) Patentinhaber: Evonik Operations GmbH, 45128 Essen (DE)
(72) Erfinder: Oelmann, Ansgar, 63571 Gelnhausen (DE); Alt, Hans Christian, 63571 Gelnhausen (DE); Dr. Becker, Franz Ulrich, 63579 Freigericht-Horbach (DE); Dr. Blümke, Wilfried, 61137 Schöneck (DE); Dr. Claes, Wilfried, 33619 Bielefeld (DE)
(74) Vertreter: Evonik Patent Association

(56) Entgegenhaltungen:
- EP-A1- 1 752 543
- EP-A2- 0 923 878
- EP-B1- 0 615 693
- WO-A2-2007/141111
- WO-A2-2008/006680
- DE-A1-102006 016 158
- DE-A1-102008 001 874
- US-A1- 2007 082 031
- US-B1- 6 238 728
- Steven R Hull ET AL: "Composition of Corn Steep Water during Steeping", Journal of Agricultural and Food Chemistry, 1 January 1996 (1996-01-01), pages 1857-1863, XP055402644, DOI: 10.1021/jf950353v Retrieved from the Internet: URL:http://pubs.acs.org/doi/pdf/10.1021/jf 950353v [retrieved on 2017-08-31]

## Beschreibung

Die Erfindung betrifft L-Aminosäure enthaltende Futtermitteladditive auf Fermentationsbrühe-Basis, die eine oberflächenaktive Substanz enthalten und aus denen die Biomasse teilweise oder vollständig entfernt wurde sowie Verfahren zu ihrer Herstellung und wird durch die Ansprüche definiert.

### Stand der Technik

Tierfuttermittel werden mit einzelnen Aminosäuren entsprechend dem Bedarf der Tiere supplementiert. Zur Supplementierung von Tierfuttermitteln, z.B. mit L-Lysin, wird bisher weit überwiegend das L-Lysin-monohydrochlorid mit einem L-Lysin-Gehalt von ca. 80 % eingesetzt. Da das L-Lysin durch Fermentation hergestellt wird, muss es zur Herstellung des Monohydrochlorids zunächst einmal von allen übrigen Bestandteilen der rohen Fermentationsbrühe in aufwendigen Verfahrensschritten abgetrennt, dann in das Monohydrochlorid umgewandelt und letzteres zur Kristallisation gebracht werden. Dabei fällt eine große Anzahl von Nebenprodukten und die zur Aufarbeitung notwendigen Reagenzien als Abfall an. Da eine hohe Reinheit des Tierfuttermittelsupplements nicht immer notwendig ist und zudem in den Nebenprodukten der Fermentation oft noch nutritiv wirksame Wertstoffe enthalten sind, hat es daher in der Vergangenheit nicht an Versuchen gefehlt, die aufwendige Herstellung von Futter-Aminosäuren, insbesondere von reinem L-Lysin-Monohydrochlorid, zu vermeiden und die rohe Fermentationsbrühe kostengünstiger in ein festes Tierfuttermittel zu überführen.

Als gravierender Nachteil hat sich die komplexe Zusammensetzung solcher Medien erwiesen, denn diese sind generell nur schlecht zu trocknen, dann hygroskopisch, praktisch nicht rieselfähig, verklumpungsgefährdet, und für die technisch anspruchsvolle Verarbeitung in Mischfutterwerken nicht geeignet. Dies trifft vor allem auf L-Lysin enthaltende Fermentationsprodukte zu. Die einfache Entwässerung der rohen Fermentationsbrühe durch Sprühtrocknung führte zu einem staubigen, stark hygroskopischen und nach kurzer Lagerzeit klumpigen Konzentrat, das in dieser Form nicht als Tierfuttermittel eingesetzt werden kann.

Die EP 0 533 039 betrifft Verfahren zur Herstellung eines Aminosäure-Tierfuttermittelsupplements auf Fermentationsbrühebasis, wobei das Supplement direkt durch Sprühtrocknung aus der Fermentationsbrühe gewonnen werden kann. Hierzu wird bei einer Variante ein Teil der Biomasse vor der Sprühtrocknung abgetrennt.

Aus der GB 1 439 121 sind ca. 20 Gew.-% L-Lysin enthaltende feste Konzentrate bekannt, in der auch L-Lysin-haltige Fermentationsbrühen mit einem pH-Wert von 4,5 und Zusatz von Natriumbisulfit beschrieben werden.

In der EP 0 615 693 wird ein Verfahren zur Herstellung eines Tierfuttermittel-Additives auf Fermentationsbrühe-Basis offenbart, bei dem man die Fermentationsbrühe, ggf. nach Entfernung eines Teils der Inhaltsstoffe, zu einem Feinkorn, das zu mind. 70 Gew.-% eine maximale Partikelgröße von 100 µm hat, sprühtrocknet, und dass man dieses Feinkorn in einer zweiten Stufe zu einem Granulat aufbaut, das zu mind. 30 Gew.-% das Feinkorn enthält.

Gemäß GB 1 439 728 wird ein L-Lysin enthaltendes Konzentrat aus einer Fermentationsbrühe hergestellt, die man vor der Aufkonzentration mit HCl auf einen pH von ca. 6,4 ansäuert und der man zur Stabilisierung Bisulfit zusetzt. Nach der Eindampfung wird weiter auf einen pH-Wert von 4,0 angesäuert und das gewünschte Produkt durch Sprühtrocknung erhalten.

Die EP 1 331 220 betrifft granulierte Futtermitteladditive, die L-Lysin als Hauptkomponente enthalten. Dort wurde gefunden, dass die Menge der Gegenionen für das Lysin, wie z.B. die der Sulfationen, verringert werden kann, indem man während der Fermentation entstehendes Hydrogencarbonat und/oder Carbonat als Gegenion benutzt. Insgesamt wird ein Anionen/Lysin-Verhältnis von 0,68 bis 0,95 beschrieben.

Die Verringerung der Gegenionen wie z. B. Sulfat im L-Lysin enthaltenden Produkt soll zu einer Verbesserung der hygroskopischen Eigenschaften und der Verbackungsneigung führen.

Die WO 2007/141111 offenbart ein Verfahren zur Herstellung eines L-Lysin enthaltenden Futtermitteladditives, enthaltend die Schritte der Fermentation eines L-Lysin produzierenden coryneformen Bakteriums, anschließendes Zusetzen von Ammoniumsulfat, Absenken des pH-Wertes durch Zugabe von Schwefelsäure auf 4,9 bis 5,2, wobei insgesamt ein Sulfat/L-Lysin-Verhältnis von 0,85 bis 1,2 in der Brühe eingestellt wird, Aufkonzentration und Trocknung, bevorzugt Granulation, unter Erhalt eines Produktes mit einem L-Lysin-Gehalt von 10 bis 70 Gew.-% bestimmt als Lysinbase, bezogen auf die Gesamtmenge.

In EP 0809940 wird beschrieben, dass die von der Fermentation stammende Biomasse bei der Granulation von Lysinsulfat positive Einflüsse auf die Granulierbarkeit sowie das Lager- und Fließverhalten des Produktes hat. Vorteilhaft ist daher eine vollständige oder teilweise Abtrennung der Biomasse um den Wirkstoffgehalt zu erhöhen. Nachteilig ist aber, dass hier nur in Grenzen ein Wirkstoffgehalt des Lysinsulfates einstellbar ist. Bei der Herstellung eines aminosäurehaltigen Futtermitteladditives wie in EP 0809940 beschrieben, wird die aus der Fermentation stammende Biomasse komplett oder teilweise im Produkt belassen. Reines Lysinsulfat ist aufgrund starker Verklebungsneingung nicht granulierbar.

Aufgabe der vorliegenden Erfindung war es daher, eine besser verarbeitbare, insbesondere besser granulierbare, biomassearme Fermentationsbrühe bereitzustellen sowie ein Verfahren bereitzustellen, das die Überführung einer L-Aminosäure, insbesondere L-Lysin, enthaltenden Fermentationsbrühe in ein besser verarbeitbares Futtermitteladditiv erlaubt. Das Verfahren sollte insbesondere ein Futtermitteadditiv mit besseren Produktspezifikationen, insbesondere hinsichtlich Korngröße, Schüttdichte, Lagerstabilität, Fließfähigkeit und/oder Handhabbarkeit, zur Verfügung stellen.

Aufgabe der vorliegenden Erfindung war es insbesondere auch, eine besser verarbeitbare, insbesondere besser granulierbare, biomassearme Lysinsulfat-haltige Fermentationsbrühe bereitzustellen sowie ein Verfahren zur Verfügung zu stellen, das die Herstellung eines biomassearmen Lysinsulfat-haltigen Futtermitteladditvs mit verbesserten Produktspezifikationen erlaubt.

Gelöst wird die erfindungsgemäße Aufgabe durch ein Verfahren, in welchem zum einen die Biomasse aus der Fermentationsbrühe teilweise oder vollständig entfernt wird und andererseits vor der Trocknung eine oberflächenaktive Substanz zu der Fermentationsbrühe hinzugegeben wird.

Überraschenderweise wurde zusätzlich gefunden, dass der verringerte Biomassegehalt in Kombination mit der oberflächenaktiven Komponente gleichzeitig zu einer erhöhten Granulat- bzw. Partikeldichte führt. Dies führt im Wesentlichen zu einem kompakteren Korn und einer erhöhten Schüttdichte. So kann durch eine gezielte Biomassereduktion und Substitution der Biomasse durch eine oberflächenaktive Substanz ein spezifikationsgerechtes Produkt mit erhöhter Schüttdichte eingestellt werden.

### Gegenstand der vorliegenden Erfindung

Gegenstand der vorliegenden Erfindung ist daher ein Verfahren zur Herstellung eines Futtermitteladditivs, dadurch gekennzeichnet, dass man eine L-Aminosäure enthaltende Fermentationsbrühe, die einen Wassergehalt von 35 bis 75 Gew.-% und einen Gehalt an oberflächenaktiver Substanz von 0,1 bis 20 Gew.-% aufweist und aus welcher die Biomasse teilweise oder vollständig entfernt wurde, durch Trocknung in eine partikuläre Zusammensetzung überführt, wobei mindestens eine der folgenden oberflächenaktiven Substanzen in folgender Menge enthalten ist: Maisquellwasser in einer Menge von 1 bis 10 Gew.-%, wobei das Maisquellwasser eine Trockenmasse von mindestens 40 Gew.-% und einen Restzuckergehalt von höchstens 2 Gew.-% aufweist; Phospholipide, Polyglykole sowie Mischungen davon jeweils in einer Menge von 0,1 bis 5 Gew.-%.

Gegenstand der vorliegenden Erfindung ist daher ebenso ein Verfahren zur Herstellung eines L-Aminosäure enthaltenden Futtermitteladditivs auf Fermentationsbrühe-Basis, folgende Schritte umfassend:
a) Bereitstellung einer L-Aminosäure enthaltenden Fermentationsbrühe;
b) teilweise oder vollständige Entfernung der Biomasse aus der Fermentationsbrühe;
c) Zugabe von oberflächenaktiver Substanz zu der Fermentationsbrühe nach Beendigung der Fermentation und vor Beginn der Trocknung, so dass die oberflächenaktive Substanz vor Beginn der Trocknung in der Fermentationsbrühe in einer Menge von 0,025 bis 20 Gew.-% enthalten ist;
d) Trocknung des erhaltenen Gemisches zu einer partikulären Zusammensetzung, wobei vorzugsweise ein Granulat erhalten wird;
e) gegebenenfalls Beschichten der erhaltenen Partikel mit einem genießbaren Öl, wobei Partikel erhalten werden, die ganz oder teilweise mit dem genießbaren Öl beschichtet sind.

Die L-Aminosäure enthaltende Fermentationsbrühe wird vorzugsweise erhalten durch Fermentation eines L-Aminosäure produzierenden Mikroorganismus in einem wässrigen Nährmedium unter aeroben Bedingungen. Erfindungsgemäß bevorzugte Fermentationsverfahren werden weiter unten näher erläutert.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten Aminosäure sicherstellen.

Bei Abschluss der Fermentation enthält die entstandene Fermentationsbrühe dementsprechend die infolge der Vermehrung der Zellen des Mikroorganismus (z.B. coryneformes Bakterium) entstandene Biomasse (= Zellmasse) des Mikroorganismus und die im Laufe der Fermentation gebildete L-Aminosäure (insbesondere L-Lysin), die im Laufe der Fermentation gebildeten organischen Nebenprodukte und die durch die Fermentation nicht verbrauchten Bestandteile des/der eingesetzten Fermentationsmediums/ Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukten gehören Stoffe, die von den bei der Fermentation eingesetzten Mikroorganismen gegebenenfalls neben dem Zielprodukt erzeugt und gegebenenfalls ausgeschieden werden. Hierzu zählen andere L-Aminosäuren, die im Vergleich zur erwünschten L-Aminosäure (insbesondere L-Lysin) weniger als 30 %, 20 % oder 10 % ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben einen L-Aminosäuregehalt (insbesondere L-Lysingehalt) von 40 g/kg bis 180 g/kg oder 50 g/kg bis 150 g/kg. Der Gehalt an Biomasse (als getrocknete Biomasse) beträgt im Allgemeinen 20 bis 50 g/kg in der Fermentationsbrühe, bei Biomasse-armer Fermentation kann der Biomasse-Gehalt jedoch auch darunter liegen.

Die Fermentationsbrühe umfasst vorzugsweise eine L-Aminosäure ausgewählt aus L-Lysin, L-Methionin, L-Threonin, L-Valin oder L-Tryptophan. Besonders bevorzugt enthält sie die L-Aminosäure L-Lysin.

In einer erfindungsgemäß besonders bevorzugten Ausführungsform handelt es sich bei der L-Aminosäure um L-Lysin, wobei das Verfahren einen zusätzlichen Verfahrensschritt umfasst, der vor Beginn der Trocknung erfolgt, in welchem Ammoniumsulfat und/oder Schwefelsäure zu der Fermentationsbrühe hinzugegeben wird, um ein Sulfat/L-Aminosäure-Verhältnis von mindestens 0,5, vorzugsweise 0,85 bis 1,2, einzustellen. Diese besonders bevorzugte Ausführungsform wird weiter unten näher ausgeführt.

Die erfindungsgemäß eingesetzte Fermentationsbrühe weist nach Beendigung der Fermentation vorzugsweise folgende Eigenschaften auf:
a) Biomasse-Gehalt von 1 bis 5 Gew.-%, vorzugsweise 2 bis 4,5 Gew.-%, besonders bevorzugt 2,5 bis 3,5 Gew.-%,
b) Gehalt an L-Aminosäure, vorzugsweise L-Lysin, (als Aminosäurebase) von 5 bis 20 Gew.-%,
c) Feststoffgehalt (inklusive Biomasse) von 10 bis 30 Gew.-%, bevorzugt 15 bis 25 Gew.-%,
d) Gew.-%-Verhältnis von Sulfat zu Lysin von 0,8 bis 1,2,
e) pH-Wert von 3,5 bis 7,0, bevorzugt von 4,0 bis 5,0.

Die Herstellung einer solchen Fermentationsbrühe wird weiter unten beschrieben.

Unter "Feststoffgehalt" ist erfindungsgemäß die Masse zu verstehen, die bei vollständigem Entzug der Flüssigkeit zurückbleibt. Zu dieser Trockenmasse gehören neben gegebenenfalls suspendierten Substanzen (wie der Biomasse) auch gelöste Substanzen, die erst bei der Trocknung auskristallisieren oder ausfällen. Der Feststoffgehalt ist insofern komplementär zum Wasser- bzw. Feuchtigkeitsgehalt.

Die Fermentationsbrühe besitzt vor Beginn der Trocknung vorzugsweise einen Wassergehalt von 35 bis 70 Gew.-%, besonders bevorzugt 35 bis 50 Gew.-%. Die Einstellung dieses Wassergehalts kann, sofern erforderlich, insbesondere durch Eindampfen der Fermentationsbrühe, beispielsweise mit Hilfe eines Rotationsverdampfers, eines Dünnschichtverdampfers oder eines Fallfilmverdampfers, durch Umkehrosmose oder Nanofiltration erfolgen. Durch das Einengen erhöht sich entsprechend auch der Gehalt an gegebenenfalls verbliebener Biomasse, der L-Aminosäure-Gehalt und der Gehalt an verbliebenem Feststoff in der Fermentationsbrühe.

Erfindungsgemäß wird vor Beginn der Trocknung vorzugsweise mindestens 30 Gew.-%, besonders bevorzugt mindestens 50 Gew.-%, insbesondere mindestens 70 Gew.-%, vor allem mindestens 90 Gew.-%, der Biomasse aus der Fermentationsbrühe entfernt. Dies kann vor oder nach der zuvor beschriebenen Einstellung des Wassergehalts erfolgen.

Die Entfernung der Biomasse kann hierbei insbesondere durch Zentrifugation, Filtration oder Dekantieren oder durch Kombinationen dieser Verfahren erfolgen. In einer erfindungsgemäß bevorzugten Ausführungsform erfolgt die Entfernung der Biomasse durch Ultrafiltration.

Die in der Fermentationsbrühe gelösten organischen Nebenprodukte und die gelösten nicht verbrauchten Bestandteile des Fermentationsmediums (Einsatzstoffe) bleiben mindestens teilweise (> 0 %), bevorzugt zu mindestens 25 %, besonders bevorzugt zu mindestens 50 % und ganz besonders bevorzugt zu mindestens 75 % im Produkt. Gegebenenfalls bleiben diese auch vollständig (100 %) oder nahezu vollständig das heißt > 95 % oder > 98 % im Produkt. In diesem Sinne bedeutet der Begriff "auf Fermentationsbrühe-Basis", dass das Produkt mindestens einen Teil der Bestandteile der Fermentationsbrühe enthält.

Die "oberflächenaktive Substanz" im Sinne der vorliegenden Anmeldung kann ein Reinstoff sein, der ausschließlich aus einer oberflächenaktiven Verbindung besteht. Es kann sich aber auch um ein Gemisch unterschiedlicher oberflächenaktiver Verbindungen handeln. Unter "oberflächenaktiver Substanz" wird erfindungsgemäß jedoch auch eine Komponente verstanden, die eine oberflächenaktive Verbindung oder eine Mischung unterschiedlicher oberflächenaktiver Verbindungen in einer signifikanten Menge enthält. Die oberflächenaktive Verbindung(en) ist/sind hierbei in der Komponente vorzugsweise in einer Menge von mindestens 3 Gew.-%, insbesondere mindestens 5 Gew.-%, besonders bevorzugt mindestens 10 Gew.-% enthalten. In einer bevorzugten Ausführungsform ist/sind die oberflächenaktive(n) Verbindung(en) in der Komponente zu mindestens 20 Gew.-%, vorzugsweise zu mindestens 25 Gew.-%, enthalten.

Die oberflächenaktive Substanz ist erfindungsgemäß vorzugsweise ausgewählt aus der Gruppe bestehend aus Maisquellwasser, Lipiden, Antischaummitteln und Tensiden sowie Mischungen davon.

Das Antischaummittel ist vorzugsweise ausgewählt aus Polysiloxanderivaten, Mono- und Polyglykolen, Phospholipiden sowie Fettsäure-Glyceriden.

Bei dem Polysiloxanderivat kann es sich insbesondere um ein Polyalkylsiloxan, vor allem um ein Polydimethylsiloxan, handeln.

Bei dem Polyglykol handelt es sich vorzugsweise um ein Polymer aus Oxyethylen- und/oder Oxypropylen-Einheiten, vorzugsweise um ein Mischpolymer aus Oxyethylen- und Oxypropylen-Einheiten, oder um eine Verbindung, die Oxyethylen- und/oder Oxypropylen-Einheiten umfasst, wie beispielsweise ein Fettsäurealkylpolyglycolester.

Bei dem Phospholipid handelt es sich vorzugsweise um ein Phosphatidylcholin (Lecithin).

Bei dem Fettsäure-Glycerid kann es sich insbesondere um ein Mono- oder Diglycerid handeln, vor allem um ein Mono- oder Diglycerid, bei welchem der Säure-Rest ausgewählt ist aus Essigsäure, Milchsäure, Citronensäure, Weinsäure und Mischungen davon.

Das erfindungsgemäß eingesetzte Maisquellwasser besitzt vorzugsweise eine Trockenmasse von mindestens 40 Gew.-%, vorzugsweise 45 bis 55 Gew.-%, und weist vorzugsweise einen Restzuckergehalt von höchstens 2 Gew.-% auf. Maisquellwasser enthält als oberflächenaktiven Bestandteil Phosphatidylcholin.

Das erfindungsgemäß einsetzbare Lipid ist vorzugsweise ausgewählt aus Mineralölen, Pflanzenölen und Mischungen davon. Besonders bevorzugt wird als Öl Sojabohnenöl, Olivenöl, Silikonöl oder Mischungen davon eingesetzt.

In einer erfindungsgemäß besonders bevorzugten Ausführungsform wird als oberflächenaktive Substanz Phosphatidylcholin oder eine Phosphatidylcholin-haltige Komponente, vorzugsweise Maisquellwasser, eingesetzt.

Die oberflächenaktive Substanz wird in einer bevorzugten Ausführungsform der Fermentationsbrühe nach Beendigung der Fermentation und vor Beginn der Trocknung hinzugegeben.

Alternativ kann die oberflächenaktive Substanz gegebenenfalls der Fermentationsbrühe bereits im Laufe der Fermentation hinzugegeben werden.

Alternativ ist es ebenso möglich, dass die oberflächenaktive Substanz bereits vor Beginn der Fermentation im Fermentationsmedium enthalten ist.

In einer erfindungsgemäß besonders bevorzugten Ausführungsform ist oberflächenaktive Substanz bereits vor Beendigung der Fermentation in der Fermentationsbrühe enthalten und weitere oberflächenaktive Substanz wird nach Beendigung der Fermentation der Fermentationsbrühe hinzugegeben.

Die oberflächenaktive Substanz ist in der Fermentationsbrühe vor Beginn der Trocknung vorzugsweise in einer Menge von 0,025 bis 20 Gew.-%, 0,1 bis 20 Gew.-%, 0,2 bis 20 Gew.-%, 0,5 bis 20 Gew.-% oder 1 bis 20 Gew.-% enthalten. Bevorzugte Bereiche sind hierbei 0,2 bis 15 Gew.-%, 0,3 bis 15 Gew.-%, 0,5 bis 15 Gew.-% sowie 1 bis 10 Gew.-%.

Sofern als oberflächenaktive Substanz ein Polyglykol, insbesondere ein Fettsäurealkylpolyglycolester, oder ein Phospholipid, insbesondere ein Lecithin, oder Mischungen davon, eingesetzt werden, wird vorzugsweise eine Konzentration an oberflächenaktiver Substanz von 0,1 bis 5 Gew.-%, insbesondere 0,2 bis 4 Gew.-%, vorzugsweise 0,25 bis 2 Gew.-%, eingestellt.

Diese Einsatzmenge an oberflächenaktiver Substanz ist generell bevorzugt, sofern es sich bei der oberflächenaktiven Substanz um eine Komponente handelt, die mindestens 50 Gew.-%, insbesondere mindestens 70 Gew.-%, an oberflächenaktiven Verbindungen enthält.

Sofern als oberflächenaktive Substanz Maisquellwasser, gegebenenfalls in Kombination mit anderen oberflächenaktiven Substanzen, eingesetzt wird, wird vorzugsweise eine Konzentration an oberflächenaktiver Substanz von 0,1 bis 10 Gew.-%, insbesondere 0,5 bis 5 Gew.-%, vorzugsweise 1 bis 3 Gew.-%, eingestellt.

Diese Einsatzmenge an oberflächenaktiver Substanz ist generell bevorzugt, sofern es sich bei der oberflächenaktiven Substanz um eine Komponente handelt, die weniger als 30 Gew.-%, insbesondere 3 bis 30 oder 3 bis 20 Gew.-%, an oberflächenaktiven Verbindungen enthält.

In einer erfindungsgemäß bevorzugten Ausführungsform weist die Fermentationsbrühe vor Beginn der Trocknung folgende Eigenschaften auf:
a) Biomasse-Gehalt von maximal 4 Gew.-%, insbesondere 0 bis 4 Gew.-% oder 0,1 bis 4 Gew.-%, bevorzugt maximal 3 Gew.-%, insbesondere 0 bis 3 Gew.-% oder 0,1 bis 3 Gew.-%, besonders bevorzugt maximal 2 Gew.-%, insbesondere 0 bis 2 Gew.-% oder 0,1 bis 2 Gew.-%, vor allem maximal 1 Gew.-%, insbesondere 0 bis 1 Gew.-% oder 0,1 bis 1 Gew.-%;
b) Gehalt an L-Aminosäure, vorzugsweise L-Lysin, (als Aminosäurebase) von 12 bis 48 Gew.-%, insbesondere 20 bis 40 Gew.-%;
c) Feststoffgehalt (inklusive Biomasse) von 20 bis 60 Gew.-%, bevorzugt 30 bis 50 Gew.-%;
d) Gehalt an oberflächenaktiver Substanz von 0,025 bis 20 Gew.-%, insbesondere 0,1 bis 20 Gew.-%, vorzugsweise 0,3 bis 15 Gew.-%;
e) Gew.-%-Verhältnis von Sulfat zu L-Aminosäure, insbesondere L-Lysin, von 0,8 bis 1,2;
f) pH-Wert von 3,5 bis 7,0, bevorzugt von 4,0 bis 5,0.

Zur Einstellung einer gewünschten L-Aminosäure-Konzentration im Produkt kann je nach Anforderung zwecks Erhöhung oder Herabsetzung des L-Aminosäuregehalts ein Additiv vor Beginn der Trocknung der Fermentationsbrühe hinzugegeben werden. Das Additiv kann alternativ und/oder zusätzlich auch während des Trocknungs- oder Granulationsprozesses hinzugefügt werden.

Um den Gehalt an L-Aminosäure zu erhöhen wird vorzugsweise die betreffende L-Aminosäure in Form eines Konzentrates oder gegebenenfalls einer weitgehend reinen Substanz beziehungsweise deren Salz in flüssiger oder fester Form hinzugefügt. Um den L-Aminosäuregehalt zu reduzieren wird vorzugsweise Ammoniumsulfat hinzugefügt. Das Additiv wird, sofern eingesetzt, vorzugsweise in einer Menge von 0,1 bis 10 Gew.-%, vorzugsweise 0,1 bis 5 Gew.-%, zu der Fermentationsbrühe hinzugegeben bzw. bevorzugt in einer Menge hinzugegeben, dass sich im finalen Produkt eine L-Aminosäure-Konzentration von 40 bis 60 Gew.-%, insbesondere 45 bis 55 Gew.-%, einstellt.

Die Trocknung zu einer partikulären Zusammensetzung kann insbesondere durch Gefriertrocknung, vorzugsweise durch ein Sprühverfahren, insbesondere Sprühtrocknung oder Sprühgranulation, erfolgen.

An die erfindungsgemäß durchzuführende Trocknung der Fermentationsbrühe können sich gegebenenfalls weitere Prozessierungsschritte, insbesondere ein oder mehrere Granulierschritte, anschließen, insbesondere, wenn bei der Trocknung nicht unmittelbar ein Granulat erhalten wird.

In einer erfindungsgemäß besonders bevorzugten Ausführungsform wird die Fermentationsbrühe jedoch unmittelbar in einem Verfahrensschritt in ein Granulat überführt, so dass eine anschließende Granulation nicht erforderlich ist. Die unmittelbare Überführung in ein Granulat erfolgt vorzugsweise durch ein Sprühgranulationsverfahren, besonders bevorzugt unter Anwendung eines Sprühgranulationsverfahrens unter Einsatz einer zirkulierenden Wirbelschicht wie in der Offenlegungsschrift WO 2005/006875 beschrieben.

In der Sprühgranulation erfolgt vorzugsweise eine vollständige oder zumindest teilweise Rückführung von stromabwärts entstehendem Staub der Granulation in die Sprühgranulationskammer.

Weiterhin wird vorzugsweise die Granulationstemperatur so geregelt, dass die Eingangstemperatur 200 bis 300°C, vorzugsweise 250 bis 275°C und die Ausgangstemperatur 60 bis 100°C, vorzugsweise 70 bis 90°C beträgt.

Das erhältliche Granulat hat vorzugsweise einen L-Aminosäure-Gehalt von 40 bis 60 Gew.-%, insbesondere 45 bis 55 Gew.-%, besonders bevorzugt 48 bis 52 Gew.-%, und einen Wassergehalt (Restfeuchte) von maximal 5 Gew.-%, vorzugsweise maximal 3,5 Gew.-%.

Durch die Trocknung wird eine partikuläre Zusammensetzung erhalten, die vorzugsweise rieselfähig ist sowie feinteilig oder grobkörnig sein kann.

Das rieselfähige, feinteilige Pulver kann wiederum durch geeignete Kompaktier- oder Granulierverfahren in ein grobkörniges, gut rieselfähiges, lagerbares und weitgehend staubfreies Produkt überführt werden.

Die Granulate sind z.B. herstellbar nach den Verfahren gemäß EP-B 0 615 693 oder EP-B 0 809 940, US 5 840 358 oder WO 2005/006875 oder WO 2004/054381.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Weiterer Gegenstand der vorliegenden Erfindung sind körnige Futtermitteladditive, folgende Merkmale umfassend:
a) Gehalt an L-Aminosäure, vorzugsweise L-Lysin, von mindestens 20 Gew.-%, vorzugsweise 25 bis 60 Gew.-%, insbesondere 30 bis 60 oder 40 bis 60 Gew.-%, besonders bevorzugt 45 bis 55 Gew.-%,
b) mittlerer Korngrößendurchmesser von 60 bis 2500 µm, vorzugsweise 60 bis 1500 µm;
c) Biomassegehalt von maximal 8 Gew.-%, insbesondere 0 bis 8 Gew.-% oder 0,1 bis 8 Gew.-%, vorzugsweise maximal 6 Gew.-%, insbesondere 0 bis 6 Gew.-% oder 0,1 bis 6 Gew.-%, besonders bevorzugt von maximal 4 Gew.-%, insbesondere 0 bis 4 Gew.-% oder 0,1 bis 4 Gew.-%, vor allem maximal 3, 2 oder 1 Gew.-%, insbesondere 0 bis 3 Gew.-%, 0 bis 2 Gew.-%, 0 bis 1 Gew.-%, 0,1 bis 3 Gew.-%, 0,1 bis 2 Gew.-% oder 0,1 bis 1 Gew.-%;
d) Gehalt an oberflächenaktiver Substanz von 0,15 bis 35 Gew.-%, vorzugsweise 0,15 bis 30 Gew.-%, besonders bevorzugt 0,5 bis 15 Gew.-%, enthaltend 3 bis 25 Gew.-% Maisquellwasser oder 0,15 bis 10 Gew.-% Phospholipide, Polyglykole oder Mischungen davon, wobei das Maisquellwasser eine Trockenmasse von mindestens 40 Gew.-% und einen Restzuckergehalt von höchstens 2 Gew.-% aufweist;
e) vorzugsweise einen Wassergehalt (Restfeuchte) von maximal 4,5 Gew.-%, insbesondere maximal 3,5 Gew.-%,
f) vorzugsweise eine das Korn umgebende Schicht aus essbarem Öl.

Bei dem angegebenen mittleren Korngrößendurchmesser handelt es sich hierbei um das arithmetische Mittel.

Bevorzugte erfindungsgemäße Futtermitteladditive besitzen einen Anteil an Partikeln von >= 70, 75, 80, 90, 95, 97 Gew.-% einer Korngröße von > 63 µm bis < 2500 µm oder einen Anteil von >= 70, 75, 80, 85, 90, 95, 97 Gew.-% einer Korngröße von > 63 bis < 2000 µm oder einem Anteil von >= 70, 75, 80, 85, 90, 95, 97 Gew.-% einer Korngröße von >100 bis < 1700 µm Durchmesser. Der Anteil an Staub d.h. Partikeln mit einer Korngröße < 63 µm liegt bevorzugt bei 20 Gew.-% oder weniger, 15 Gew.-% oder weniger, 10 Gew.-% oder weniger, 5 Gew.-% oder weniger, 3 Gew.-% oder weniger, 2 Gew.-%, 0 bis 1 Gew.-%, 0,5 Gew.-% oder weniger.

Besonders bevorzugt weisen mindestens 75 Gew.-% der Partikel der erhaltenen Zusammensetzung einen Korngrößendurchmesser von > 63 µm bis < 2500 µm, vorzugsweise von > 63 µm bis < 1700 µm, insbesondere von > 63 µm bis < 2000 µm, auf, wobei vorzugsweise der Anteil der Partikel mit einem Korngrößendurchmesser von < 63 µm 20 Gew.-% oder weniger beträgt.

Das Schüttgewicht der bevorzugten Produkte beträgt im Allgemeinen 600 bis 800 kg/m³.

Die Partikelgrößenverteilung wird vorzugsweise mittels Siebanalyse in einer Luftstrahlsiebmaschine Hosokawa Alpine, Typ 200 LS-N, gemessen (Siebsatz: Maschenweiten 20, 32, 45, 63, 100, 150, 200, 250, 280, 300, 400, 500, 600, 630, 710, 800, 1000, 1180, 1400, 1600 und 2000 µm; Siebung: 3 min.).

Alternativ kann die Partikelgröße beispielsweise auch durch Laserbeugungsspektrometrie ermittelt werden. Anwendbare Methoden sind im Lehrbuch "Teilchengrößenmessung in der Laborpraxis" von R.H. Müller und R. Schuhmann, Wissenschaftliche Verlagsgesellschaft Stuttgart (1996) sowie im Lehrbuch "Introduction to Particle Technology" von M. Rhodes, Verlag Wiley & Sons (1998) beschrieben.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP-A 0 743 016) oder Stearaten.

Gegebenenfalls wird aus der erhaltenen partikulären Zusammensetzung bzw. dem erhaltenen Granulat durch Sieben, Walzen, Staubtrennung, Vermahlung oder Kombinationen davon ein Produkt mit der gewünschten Korngröße erhalten.

Erfindungsgemäße körnige Futtermitteladditive zeichnen sich vorzugsweise weiterhin dadurch aus, dass sie mit einem Öl beschichtet sind, wie beispielsweise in der WO 04/054381 beschrieben, wobei das Öl vorzugsweise ausgewählt ist aus pflanzlichem Öl (insbesondere Olivenöl, Sonnenblumenöl, Sojaöl oder Sojaöl/Lecithingemische), tierischem Öl oder Fett und von Mikroorganismen durch Fermentation erzeugtem Öl. Durch die Behandlung der Oberflächen mit den genannten Ölen erzielt man eine erhöhte Abriebfestigkeit des Produktes und eine Verringerung des Staubanteils.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken, Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C 41 00 920 beschrieben, veredelt werden.

Die Biomasse in erfindungsgemäßen Futtermitteladditiven umfasst vorzugsweise Bakterien der Gattung Corynebacterium oder der Gattung Escherichia und/oder Zelltrümmer dieser Bakterien und besteht besonders bevorzugt im Wesentlichen daraus.

Der Gehalt an L-Aminosäure in erfindungsgemäßen Futtermitteladditiven beträgt vorzugsweise mindestens 30 Gew.-%, vorzugsweise mindestens 40 Gew.-%, insbesondere 40 bis 60 Gew.-%, besonders bevorzugt 45 bis 55 Gew.-%.

Die L-Aminosäure in den erfindungsgemäßen Futtermitteladditiven ist vorzugsweise ausgewählt aus der Gruppe bestehend aus L-Lysin, L-Methionin, L-Threonin, L-Tryptophan und L-Valin sowie Mischungen davon, besonders bevorzugt handelt es sich bei der L-Aminosäure um L-Lysin.

Bei dem erfindungsgemäßen Futtermitteladditiv handelt es sich vorzugsweise um ein Futtermitteladditiv auf Fermentationsbrühe-Basis.

Die in erfindungsgemäßen körnigen Futtermitteladditiven enthaltene oberflächenaktive Substanz ist vorzugsweise ausgewählt aus den zuvor genannten oberflächenaktiven Substanzen. Das Futtermitteladditive enthält jedoch zwingend 3 bis 25 Gew.-% Maisquellwasser oder 0,15 bis 10 Gew.-% Phospholipide, Polyglykole oder Mischungen davon, wobei das Maisquellwasser eine Trockenmasse von mindestens 40 Gew.-% und enen Restzuckergehalt von höchstens 2 Gew.-% aufweist.

Sofern es sich bei der oberflächenaktiven Substanz um ein Polyglykol, insbesondere ein Fettsäurealkylpolyglycolester, oder ein Phospholipid, insbesondere ein Lecithin, oder Mischungen davon, handelt, ist die oberflächenaktive Substanz im Futtermitteladditiv in einer Menge von 0,15 bis 10 Gew.-%, insbesondere 0,3 bis 6 Gew.-%, besonders bevorzugt 0,4 bis 4 Gew.-%, enthalten.

Sofern es sich bei der oberflächenaktiven Substanz um Maisquellwasser, gegebenenfalls in Kombination mit anderen oberflächenaktiven Substanzen, handelt, ist die oberflächenaktive Substanz im Futtermitteladditiv in einer Menge von 3 bis 25 Gew.-%, besonders bevorzugt 6 bis 20 Gew.-%, enthalten.

Die Verteilung der oberflächenaktiven Substanz im Korn ist vorzugsweise homogen, wobei unter "homogen" zu verstehen ist, dass zwischen zwei beliebigen Fraktionen des Korns kein großer Konzentrationsunterschied der oberflächenaktiven Substanz anzutreffen ist.

Vorzugsweise beträgt die Abweichung der Menge der oberflächenaktiven Substanz in zwei beliebigen Fraktionen des Korns, bei denen es sich beispielsweise um beliebige Kuben mit einem Volumen von 10 x 10 µm handeln kann, maximal 30 %, vorzugsweise maximal 25 oder 20 %, besonders bevorzugt maximal 10, 5 oder 3 %.

Die homogene Verteilung der oberflächenaktiven Substanz wird durch die Weise der Herstellung des Futtermitteladditivs sichergestellt.

Die Partikeldichte des Futtermitteladditivs beträgt vorzugsweise mindestens 1,20 g/cm³, besonders bevorzugt 1,20 bis 1,30 g/cm³, vor allem 1,20 bis 1,26 g/cm³.

Die Schüttdichte des Futtermitteladditivs beträgt vorzugsweise mindestens 600 kg/m³, insbesondere 600 bis 800 kg/m³.

Die Schüttdichte wird vorzugsweise wie folgt bestimmt: Ein leerer Standzylinder (250 ml Volumen) wird auf einer Waage platziert, mit dem körnigen Produkt befüllt und anschließend das Gewicht pro Volumeneinheit ermittelt.

Zur Ermittlung der Partikeldichte werden die Leerräume im Standzylinder mit Methanol aufgefüllt. Das Lückenvolumen kann so aufgrund der Gewichtszunahme und der bekannten Dichte des Methanols (0,7918 g/ml) ermittelt werden. Aus der Differenz zwischen Gesamtvolumen und Volumen des Methanols ergibt sich so das Volumen der Partikel. Die Partikeldichte ergibt sich dann dadurch, indem man das zuvor ermittelte Gewicht der Partikel nicht auf das Gesamtvolumen des Messzylinders bezieht, sondern auf das ermittelte Volumen der Partikel.

Alternativ kann die Partikeldichte auch unter Verwendung eines Pyknometers ermittelt werden. Die Bestimmung der Partikeldichte erfolgt hierbei durch Gasverdrängung. Vorzugsweise werden inerte Gase wie Helium oder Stickstoff als Verdrängungsmedium eingesetzt. Ein im Handel erhältliches Pyknometer stellt hierbei beispielsweise das Helium-Pyknometer AccuPyc 1340 (mimetrics) dar.

Erfindungsgemäße körnige Futtermitteladditive zeichnen sich vorzugsweise dadurch aus, dass sie die L-Aminosäure L-Lysin enthalten, wobei diese vorzugsweise zumindest teilweise als Sulfatsalz vorliegt, wobei das molare Verhältnis von Sulfat zu L-Lysin vorzugsweise mindestens 0,5, besonders bevorzugt 0,8 bis 1,2 beträgt.

In der bevorzugten Ausführungsform, in welcher es sich bei der L-Aminosäure um L-Lysin handelt, haben erfindungsgemäße Futtermitteladditive vorzugsweise einen pH-Wert von 3,5 bis 6,5, insbesondere 4,0 bis 5,0, bevorzugt 4,2 bis 4,8, gemessen in wässriger Suspension. Für die pH-Messung wird eine 10 Gew.-%ige Suspension in entionisiertem Wasser hergestellt und der pH bei 25°C mit einer pH-Elektrode gemessen. Der Messwert stellt sich nach ca. 1 Minute konstant ein.

Vorzugsweise liegt der Wassergehalt erfindungsgemäßer Futtermitteladditive zwischen 0,1 Gew.-% und maximal 5 Gew.-%. Der Wassergehalt beträgt bevorzugt maximal 4% Gew.-%, besonders bevorzugt maximal 3% Gew.-% und ganz besonders bevorzugt maximal 2,5 Gew.-%. Wassergehalte von maximal 2 Gew.-% sind ebenfalls möglich.

Erfindungsgemäße Futtermitteladditive zeichnen sich vorzugsweise weiterhin dadurch aus, dass sie eine sehr kompakte Struktur aufweisen, wobei unter "kompakter Struktur" zu verstehen ist, dass sie relativ wenig Hohlräume aufweisen. Dies ist nicht zuletzt auf den Einsatz der oberflächenaktiven Substanz zurückzuführen. Erfindungsgemäße Futtermitteladditive zeichnen sich vorzugsweise dadurch aus, dass sie weniger als 25 Vol.-%, insbesondere weniger als 20 Vol.-%, besonders bevorzugt weniger als 15 Vol.-%, vor allem weniger als 10 Vol.-%, Hohlräume aufweisen.

Weiterer Gegenstand der vorliegenden Erfindung ist die Verwendung eines erfindungsgemäßen körnigen Futtermitteladditivs zur Herstellung von Futtermitteln.

### Herstellung der L-Aminosäure enthaltenden Fermentationsbrühe

Die fermentative Herstellung von L-Aminosäuren, wie L-Lysin, L-Methionin, L-Threonin, L-Tryptophan, L-Valin, insbesondere L-Lysin wird durch fermentative Kultivierung eines Aminosäure überproduzierenden Bakterienstammes erreicht. Die Fermentation erfolgt bevorzugt mit coryneformen Bakterien, insbesondere der Gattung *Corynebacterium,* besonders bevorzugt der Art *Corynebacterium glutamicum,* und /oder der Gattung *Escherichia,* besonders bevorzugt der Art *Escherichia coli,* durch ein sogenanntes fed-batch Verfahren (Zulaufverfahren). Alternativ kann auch kontinuierlich oder diskontinuierlich im batch-Verfahren (Satzkultivierung) oder repeated-fed-batch Verfahren (repetitives Zulaufverfahren) die Fermentation mit dem Ziel der Produktion von L-Aminosäuren (insbesondere L-Lysin) durchgeführt werden. Das verwendete Fermentationsmedium ist an die Ansprüche der jeweiligen Produktionsstämme optimiert. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/ Wiesbaden, 1994)) verfügbar.

Das zu verwendende Kulturmedium beziehungsweise Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology", der American Society for Bacteriology (Washington D. C, USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnussöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniak, Ammoniumsulfat, Ammoniumphosphat, Ammoniumcarbonat und Ammoniumnitrat, bevorzugt Ammoniak oder Ammoniumsulfat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze beispielsweise in Form von Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren, beispielsweise Homoserin, und Vitamine, beispielsweise Thiamin, Biotin oder Pantothensäure, zusätzlich zu den oben genannten Stoffen eingesetzt werden. Dem Kulturmedium können überdies geeignete Vorstufen der jeweiligen Aminosäure zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser bevorzugt Ammoniak oder Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt.

Zur Kontrolle der Schaumentwicklung können Antischäummittel, wie zum Beispiel Fettsäurepolyglykolester eingesetzt werden. Zur Aufrechterhaltung der Stabilität von Plasmiden können dem Medium gegebenenfalls geeignete selektiv wirkende Stoffe, wie zum Beispiel Antibiotika hinzugefugt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich.

Gegebenenfalls wird die Fermentation bei Überdruck, beispielsweise bei einem Druck von 0,03 bis 0,2 MPa, gefahren. Die Temperatur der Kultur liegt normalerweise bei 20 °C bis 45 °C und vorzugsweise bei 25 °C bis 40 °C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich. Um entsprechend große Produktionsfermentervolumina von mehreren hundert Kubikmetern zu fermentieren sind mehrere vorgeschaltete Anzuchtfermenterschritte mit sukzessive zunehmendem Kesselvolumen notwendig.

Beispiele für geeignete Fermentationsmedien finden sich unter anderem in den Patentschriften US 5,770,409, US 5,840,551 und US 5,990,350, US 5,275,940 oder US 4,275,157. Weitere Beispiele für Fermentationsmedien finden sich bei Ozaki und Shiio (Agricultural and Biological Chemistry 47(7), 1569-1576, 1983) und Shiio et al. (Agricultural and Biological Chemistry 48(6), 1551-1558, 1984). Methoden zur Bestimmung von L-Lysin und anderer L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Die auf diese Weise hergestellte Fermentationsbrühe wird anschließend erfindungsgemäß weiterverarbeitet.

Die Biomasse beziehungsweise die Biomasse enthaltene Fermentationsbrühe wird vorzugsweise während eines geeigneten Verfahrensschrittes thermisch inaktiviert, bevor die Biomasse vollständig oder teilweise entfernt wird.

### Bevorzugte Verfahrensdurchführung bei der Produktion von L-Lysin

Im Falle, dass die produzierte L-Aminosäure L-Lysin ist, wird, wie bereits zuvor erwähnt, vorzugsweise ein zusätzlicher Verfahrensschritt durchgeführt, der vor Beginn der Trocknung erfolgt, in welchem Ammoniumsulfat und/oder Schwefelsäure zu der Fermentationsbrühe hinzugegeben werden, um ein molares Sulfat/L-Aminosäure-Verhältnis von mindestens 0,5 einzustellen. Das molare Sulfat/L-Lysin-Verhältnis beträgt hierbei bevorzugt mindestens 0,6, 0,8, 0,9 oder 0,95, insbesondere 0,85 bis 1,2 , vorzugsweise 0,9 bis 1,1, besonders bevorzugt >0,95 bis <1,1.

Das molare Sulfat/L-Lysin-Verhältnis V wird nach der Formel: V = 2 x [SO4²⁻] / [L-Lysin] berechnet.

Diese Formel berücksichtigt die Tatsache, dass das Sulfat-Anion zweiwertig ist. Ein Verhältnis V = 1 bedeutet, dass ein stöchiometrisch zusammengesetztes Lys₂(SO₄) vorliegt, während bei einem Verhältnis von V = 0,9 ein 10%iger Sulfatunterschuss und bei einem Verhältnis von V = 1,1 ein 10%iger Sulfatüberschuss gefunden wird.

Es ist alternativ möglich, die Fermentation in Gegenwart einer solchen Menge von Ammoniumsulfat durchzuführen, dass nach Beendigung der Fermentation bereits ein Sulfat/L-Aminosäure-Verhältnis vorliegt, das im erfindungsgemäß bevorzugten Bereich liegt. In diesem Fall kann auf den zusätzlichen Verfahrensschritt verzichtet werden.

Schließlich kann die Brühe auch mit bevorzugt Natriumbisulfit (Natriumhydrogensulfit) oder einem anderen Salz, beispielsweise Ammonium-, Alkali- oder Erdalkalisalz der schwefligen Säure, versetzt werden, was zur Stabilisierung und Aufhellung des Produktes führt.

In diesem Sinne umfasst ein erfindungsgemäß besonders bevorzugtes Verfahren folgende Schritte:
- Bereitstellung einer L-Lysin enthaltenden Fermentationsbrühe;
- teilweise oder vollständige Entfernung der Biomasse, vorzugsweise Entfernung von mindestens 50 oder 60 Gew.-%, besonders bevorzugt von mindestens 90 oder 95 Gew.-% der Biomasse;
- gegebenenfalls Messen des Verhältnisses von Sulfat zu L-Lysin;
- anschließend gegebenenfalls Zusetzen von Ammoniumsulfat und/oder Maisquellwasser;
- gegebenenfalls Zusetzen von Schwefelsäure;
- Einstellen des pH-Wertes durch Zugabe von Schwefelsäure auf 4,0 bis 6,5, insbesondere 4,9 bis 5,1, wobei durch die Zugabe der Sulfat-haltigen Verbindung in den vorgenannten Schritten ein Sulfat/L-Aminosäure-Verhältnis von 0,85 bis 1,2, bevorzugt 0,9 bis 1,0 besonders bevorzugt >0,9 bis <0,95 in der Brühe eingestellt wird;
- gegebenenfalls Einengung der Fermentationsbrühe auf einen Wassergehalt von 35 bis 70 Gew.-%, insbesondere 35 bis 50 Gew.-%;
- Zugabe einer oberflächenaktiven Komponente, so dass sich ein Gehalt an oberflächenaktiver Komponente von 0,025 bis 20 Gew.-%, insbesondere 0,1 bis 20 Gew.-%, vorzugsweise 0,2 bis 15 Gew.-%, besonders bevorzugt 0,3 bis 10 Gew.-%, einstellt;
- Trocknung des Gemisches zu einer partikulären Zusammensetzung, vorzugsweise durch Sprühgranulation;
- Gegebenenfalls Beschichten der Partikel nach Schritt mit einem genießbaren Öl, wobei Partikel erhalten werden, die ganz oder teilweise mit dem genießbaren beschichtet sind.

Unter Sulfat-haltigen Verbindungen im Sinne der oben genannten Verfahrensschritte sind insbesondere Ammoniumsulfat und Schwefelsäure gemeint. Auf diese Weise erhält man ein Produkt mit einem L-Aminosäure-Gehalt (insbesondere L-Lysin) von 10 bis 70 Gew.-% (berechnet als Aminosäure, bezogen auf die Gesamtmenge) und im Falle, dass die L-Aminosäure L-Lysin ist, liegt L-Lysin in einem molaren Sulfat/L-Lysin-Verhältnis von mindestens 0,5, bevorzugt 0,6, 0,8, 0,9, 0,95, 1,0, 1,05, 1,1, 1,2, weiter bevorzugt von 0,85 bis 1,2 , bevorzugt 0,9 bis 1,1, besonders bevorzugt >0,95 bis <1,1 vor.

Wird über die erfindungsgemäße pH-Wertabsenkung hinaus Säure zugesetzt, sind wegen der Pufferwirkung der in der Brühe enthaltenen Verbindungen erhöhte Mengen an Säure erforderlich, die dann zu einer unerwünschten Denaturierung und Auflösung der Zellen der coryneformen Bakterien führen können.

In einer erfindungsgemäßen Verfahrensvariante wird der Fermentationsbrühe eines oder mehrerer der Salze der schwefeligen Säure (Sulfite) ausgewählt aus der Gruppe Ammonium-, Alkali-, und Erdalkalisalz in einer Menge von 0,01 bis 0,5 Gew.-%, bevorzugt 0,1 bis 0,3 Gew.-%, besonders bevorzugt 0,1 bis 0,2 Gew.-%, bezogen auf die Fermentationsbrühe zugesetzt. Bevorzugt wird Alkalihydrogensulfit und besonders bevorzugt Natriumhydrogensulfit eingesetzt.

Die Sulfite, insbesondere Natriumhydrogensulfit werden bevorzugt als Lösung vor der Einengung der Fermentationsbrühe zugesetzt. Die eingesetzte Menge wird bevorzugt bei der Einstellung des Sulfat-/L-Aminosäure-Verhältnisses berücksichtigt.

Bei dem erfindungsgemäßen Verfahren zur Herstellung von L-Aminosäuren (insbesondere L-Lysin) enthaltenden Futtermitteladditiven werden solche Vorgehensweisen bevorzugt, bei denen Produkte erhalten werden, die Bestandteile der Fermentationsbrühe enthalten.

### Ausführungsbeispiele

### Beispiel 1: Einfluss von Biomasse-Gehalt und Gehalt an oberflächenaktiver Substanz auf die Granulierbarkeit der Fermentationsbrühe

Lysinsulfat enthaltende Fermentationsbrühe wurde bereitgestellt und aus dieser durch Ultrafiltration die Biomasse abgetrennt. Die dadurch erhaltenen Fraktionen (Biomassekonzentrat und biomassefreies Permeat) wurden anschließend in verschiedenen Mengenanteilen miteinander vereint, um gezielt Fermentationsbrühen mit unterschiedlichem Biomassegehalt zu erhalten. Es wurden so Fermentationsbrühen mit einem Gehalt an Biomasse von 0; 0,4; 3,1; 3,6; 7,1; 8,8; 10,4 bzw. 12,0 Gew.-% hergestellt.

Weiterhin wurden Fermentationsbrühen ohne Biomasse sowie Fermentationsbrühen mit einem Gehalt an Biomasse von 3,1 bzw. 12,0 Gew.-% mit unterschiedlichen Mengen an Maisquellwasser versetzt. Maisquellwasser enthält als oberflächenaktive Komponente Lecithin. Maisquellwasser wurde der biomassenfreien Fermentationsbrühe in Mengen von 0; 1,0; 2,3 bzw. 4,6 Gew.-% hinzugegeben, der 3,1 und 12,0 Gew.-% Biomasse enthaltenden Fermentationsbrühe in einer Menge von jeweils 2,3 Gew.-%.

Mit diesen Materialien wurde eine Wirbelschichtsprühgranulation durchgeführt. Hierzu wurden jeweils 300 g zerkleinertes Lysin-Granulat vorgelegt und mit jeweils 3800 g Fermentationsbrühe mit unterschiedlichem Gehalt an Biomasse bzw. Maisquellwasser und einer Trockenmasse von ca. 56 Gew.-% aufgranuliert.

Die Sprühgranulation wurde durchgeführt bei einer Eingangstemperatur von 150-160°C und einer Wirbelschichttemperatur von 85°C.

Im Anschluss an die Sprühgranulation wurde jeweils die Schüttdichte und die Partikeldichte bestimmt.

Die Schüttdichte wurde wie folgt bestimmt: Ein leerer Standzylinder (250 ml Volumen) wurde auf einer Waage platziert, mit dem körnigen Produkt befüllt und anschließend das Gewicht pro Volumeneinheit ermittelt.

Zur Ermittlung der Partikeldichte wurden die Leerräume im Standzylinder mit Methanol aufgefüllt. Das Lückenvolumen konnte so aufgrund der Gewichtszunahme und der bekannten Dichte des Methanols (0,7918 g/ml) ermittelt werden. Aus der Differenz zwischen Gesamtvolumen und Volumen des Methanols ergibt sich so das Volumen der Partikel. Die Partikeldichte ergibt sich dann dadurch, indem man das zuvor ermittelte Gewicht der Partikel nicht auf das Gesamtvolumen des Messzylinders bezieht, sondern auf das ermittelte Volumen der Partikel.

### Ergebnis

Es stellte sich heraus, dass alle untersuchten Mischungen granuliert werden konnten. Es wurde jedoch eine stärkere Klebeneigung mit abnehmender Biomasse beobachtet. Überraschenderweise konnte jedoch trotz der kompletten Abwesenheit von Biomasse eine Granulation erfolgen, auch wenn zur Kontrolle der Partikelgröße geeignete Maßnahmen erforderlich sein können wie z.B der Einsatz von Messerwerken in der Wirbelschicht oder eine externe Keimzugabe. Die Granulierbarkeit bei Abwesenheit von Biomasse war aber sehr erschwert.

Es wurde nun festgestellt, dass die Zugabe des Maisquellwassers zu einer deutlich geringeren Klebeneigung und damit deutlich verbesserten Granulierbarkeit der Fermentationsbrühe führt. Bessere Eigenschaften konnten hierbei mit geringeren Mengen an Maisquellwasser erzielt werden, da mit zunehmender Menge an Maisquellwasser die Hygroskopizität des Produkts zunahm, so dass sich eine Einsatzmenge an Maisquellwasser von 1 bis 3 Gew.-% als erfindungsgemäß besonders vorteilhaft hinsichtlich der Lagerstabilität herausstellte.

Überraschenderweise wurde aber darüber hinaus auch eine Abhängigkeit der Partikeldichte vom Biomassegehalt und vom Gehalt an oberflächenaktiver Substanz gefunden.

Es zeigte sich, dass mit abnehmender Biomasse die Partikeldichte zunahm und dass die Partikeldichte der biomassefreien Zubereitung aufgrund der Zugabe von Maisquellwasser kontinuierlich weiter zunahm. Dies bringt deutliche Vorteile in Verpackung und Transportkosten.

Die Ergebnisse der Messungen sind in den folgenden Tabellen dargestellt.

**Tabelle 1: Abhängigkeit der Partikeldichte und Schüttdichte vom Biomassegehalt in der Ausgangs-Fermentationsbrühe**

| Biomasse [Gew.-%] | Partikeldichte [g/l] | Schüttdichte [g/l] |
|---|---|---|
| 10,4 | 1113 | 613,2 |
| 8,8 | 1123 | 607,6 |
| 7,1 | 1118 | 620,4 |
| 3,6 | 1172 | 632,8 |
| 0,4 | 1185 | 636,4 |
| 0,0 | 1182 | 634,8 |

In der zweiten Versuchsreihe zeigte sich, dass sich die Partikeldichte nach der Entfernung von Biomasse durch die Zugabe von CSL weiter erhöht werden kann. Dieser Effekt ist auch bei Anwesenheit von Biomasse zu beobachten, jedoch weniger stark ausgeprägt. Umso höher die Mengen an verbliebener Biomasse umso geringer ist die erreichbare Partikeldichte, wie Tabelle 2 zu entnehmen.

**Tabelle 2: Abhängigkeit der Partikeldichte des Granulats vom Gehalt an Biomasse und Maisquellwasser (CSL) in der Ausgangs-Fermentationsbrühe**

| Biomasse [Gew.-%] | CSL-Gehalt [Gew.-%] | Partikeldichte [g/l] |
|---|---|---|
| 0,0 | 0,0 | 1212 |
| 0,0 | 1,0 | 1217 |
| 0,0 | 2,3 | 1233 |
| 0,0 | 4,6 | 1250 |
| 3,1 | 2,3 | 1177 |
| 12,0 | 2,3 | 1168 |

## Patentansprüche

1. Verfahren zur Herstellung eines Futtermitteladditivs, **dadurch gekennzeichnet, dass** man eine L-Aminosäure enthaltende Fermentationsbrühe, die einen Wassergehalt von 35 bis 75 Gew.-% und einen Gehalt an oberflächenaktiver Substanz von 0,1 bis 20 Gew.-% aufweist und aus welcher die Biomasse teilweise oder vollständig entfernt wurde, durch Trocknung in eine partikuläre Zusammensetzung überführt, wobei mindestens eine der folgenden oberflächenaktiven Substanzen in folgender Menge enthalten ist: Maisquellwasser in einer Menge von 1 bis 10 Gew.-%, wobei das Maisquellwasser eine Trockenmasse von mindestens 40 Gew.-% und einen Restzuckergehalt von höchstens 2 Gew.-% aufweist; Phospholipide, Polyglykole sowie Mischungen davon jeweils in einer Menge von 0,1 bis 5 Gew.-%.

2. Verfahren zur Herstellung eines L-Aminosäure enthaltenden Futtermitteladditivs, folgende Schritte umfassend:
a) Bereitstellung einer L-Aminosäure enthaltenden Fermentationsbrühe;
b) teilweise oder vollständige Entfernung der Biomasse aus der Fermentationsbrühe;
c) Zugabe von oberflächenaktiver Substanz zu der Fermentationsbrühe nach Beendigung der Fermentation und vor Beginn der Trocknung, so dass die oberflächenaktive Substanz vor Beginn der Trocknung in der Fermentationsbrühe in einer Menge von 0,025 bis 20 Gew.-% enthalten ist;
d) Trocknung des erhaltenen Gemischs zu einer partikulären Zusammensetzung;
e) gegebenenfalls Beschichten der Partikel mit einem genießbaren Öl, wobei Partikel erhalten werden, die ganz oder teilweise mit dem genießbaren Öl beschichtet sind.

3. Verfahren gemäß Anspruch 1 oder 2, **dadurch gekennzeichnet, dass** es sich bei der L-Aminosäure um L-Lysin, L-Methionin, L-Threonin, L-Valin oder L-Tryptophan, vorzugsweise um L-Lysin, handelt.

4. Verfahren gemäß Anspruch 1 oder 2, **dadurch gekennzeichnet, dass** es sich bei der produzierten Aminosäure um L-Lysin handelt und dass in einem Verfahrensschritt nach Beendigung der Fermentation und vor Beginn der Trocknung Ammoniumsulfat und/oder Schwefelsäure zu der Fermentationsbrühe hinzugegeben wird, um ein Sulfat/L-Aminosäure-Verhältnis von 0,85 bis 1,2 einzustellen.

5. Verfahren nach einem der vorhergehenden Ansprüche, **dadurch gekennzeichnet, dass** unter "teilweiser oder vollständiger Entfernung der Biomasse" eine Entfernung von mindestens 30 Gew.-%, vorzugsweise mindestens 50 Gew.-%, insbesondere mindestens 70 Gew.-%, besonders bevorzugt mindestens 90 Gew.-% der Biomasse zu verstehen ist, wobei die Entfernung der Biomasse vorzugsweise durch Ultrafiltration erfolgt.

6. Verfahren nach einem der vorhergehenden Ansprüche, **dadurch gekennzeichnet, dass** ein Biomassegehalt von maximal 4 Gew.-%, vorzugsweise maximal 3 Gew.-%, besonders bevorzugt maximal 2 Gew.-%, insbesondere maximal 1 Gew.-%, eingestellt wird.

7. Verfahren nach einem der vorhergehenden Ansprüche, **dadurch gekennzeichnet, dass** die oberflächenaktive Substanz vor der Trocknung in einer Menge von 0,025 bis 20 Gew.-%, insbesondere 0,1 bis 20 Gew.-%, vorzugsweise 0,3 bis 10 Gew.-%, in der Fermentationsbrühe enthalten ist.

8. Verfahren nach einem der vorhergehenden Ansprüche, **dadurch gekennzeichnet, dass** vor der Trocknung der Wassergehalt in der Fermentationsbrühe auf einen Wert von 35 bis 50 Gew.-% eingestellt wird, wobei die Einstellung des Wassergehalts vorzugsweise durch Eindampfen, Umkehrosmose oder Nanofiltration erfolgt.

9. Verfahren nach einem der vorhergehenden Ansprüche, **dadurch gekennzeichnet, dass** die in der Trocknung eingesetzte Fermentationsbrühe folgende Eigenschaften aufweist:
a) Biomasse-Gehalt von maximal 4 Gew.-%, vorzugsweise maximal 3 Gew.-%, besonders bevorzugt maximal 2 Gew.-%, vor allem maximal 1 Gew.-%;
b) Gehalt an L-Aminosäure, vorzugsweise L-Lysin, (als Aminosäurebase) von 12 bis 48 Gew.-%;
c) Feststoffgehalt (inklusive Biomasse) von 20 bis 60 Gew.-%, bevorzugt 30 bis 50 Gew.-%;
d) Gehalt an oberflächenaktiver Substanz von 0,025 bis 20 Gew.-%, vorzugsweise 0,1 bis 20 Gew.-%, insbesondere 0,3 bis 10 Gew.-%;
e) Gew.-%-Verhältnis von Sulfat zu L-Aminosäure, insbesondere L-Lysin, von 0,8 bis 1,2;
f) pH-Wert von 3,5 bis 7,0, bevorzugt von 4,0 bis 5,0.

10. Verfahren nach einem der vorhergehenden Ansprüche, **dadurch gekennzeichnet, dass** die Trocknung durch Sprühtrocknung, vorzugsweise Sprühgranulation, erfolgt, wobei bei der Sprühgranulation vorzugsweise ein Wirbelschicht-Reaktor eingesetzt wird.

11. Körniges Futtermitteladditiv, folgende Merkmale umfassend:
a) Gehalt an L-Aminosäure, vorzugsweise L-Lysin, von mindestens 20 Gew.-%, vorzugsweise 25 bis 60 Gew.-%, insbesondere 30 bis 60 oder 40 bis 60 Gew.-%, besonders bevorzugt 45 bis 55 Gew.-%,
b) mittlerer Korngrößendurchmesser von 60 bis 2500 µm, vorzugsweise 60 bis 1500 µm;
c) Biomassegehalt von maximal 8 Gew.-%, vorzugsweise maximal 6 oder 4 Gew.-%, besonders bevorzugt maximal 3, 2 oder 1 Gew.-%;
d) Gehalt an oberflächenaktiver Substanz von 0,15 bis 35 Gew.-%, vorzugsweise 0,15 bis 30 Gew.-%, besonders bevorzugt 0,5 bis 15 Gew.-%, enthaltend 3 bis 25 Gew.-% Maisquellwasser oder 0,15 bis 10 Gew.-% Phospholipide, Polyglykole oder Mischungen davon, wobei das Maisquellwasser eine Trockenmasse von mindestens 40 Gew.-% und einen Restzuckergehalt von höchstens 2 Gew.-% aufweist;
e) vorzugsweise einen Wassergehalt (Restfeuchte) von maximal 3,5 Gew.-%,
f) vorzugsweise eine das Korn umgebende Schicht aus essbarem Öl.

12. Futtermitteladditiv gemäß Anspruch 11, **dadurch gekennzeichnet, dass** es sich bei der L-Aminosäure um L-Lysin handelt, diese zumindest teilweise als Sulfatsalz vorliegt und das molare Verhältnis von Sulfat zu L-Lysin mindestens 0,5, vorzugsweise 0,8 bis 1,2, beträgt.

13. Futtermitteladditiv nach einem der vorhergehenden Ansprüche, **dadurch gekennzeichnet, dass** es weniger als 30 Vol.-%, vorzugsweise weniger als 20 Vol.-%, besonders bevorzugt weniger als 15 Vol.-% Hohlräume aufweist.

## Claims

1. Method for preparing a feed additive, **characterized in that** a fermentation broth containing L-amino acid, which has a water content of 35 to 75% by weight and a content of surface-active substance of 0.1 to 20% by weight, and from which the biomass has been partially or completely removed, is converted by drying into a particulate composition wherein at least one of the following surface-active substances are present in the following amounts: corn steep liquor in an amount of 1 to 10% by weight, wherein the corn steep liquor has a dry mass of at least 40% by weight and a residual sugar content of at most 2% by weight; phospholipids, polyglycols and mixtures thereof each in an amount of 0.1 to 5% by weight.

2. Method for preparing a feed additive containing L-amino acid, comprising the following steps:
a) providing a fermentation broth containing L-amino acid;
b) partially or completely removing the biomass from the fermentation broth;
c) adding a surface-active substance to the fermentation broth after completion of the fermentation and before the start of the drying process, such that the surface-active substance is present in the fermentation broth before the start of the drying in an amount of 0.025 to 20% by weight;
d) drying the resulting mixture to give a particulate composition;
e) optionally coating the particle with an edible oil, wherein particles are obtained which are completely or partially coated with the edible oil.

3. Method according to Claim 1 or 2, **characterized in that** the L-amino acid is L-lysine, L-methionine, L-threonine, L-valine or L-tryptophan, preferably L-lysine.

4. Method according to Claim 1 or 2, **characterized in that** the amino acid produced is L-lysine and that in a method step after completion of the fermentation and before the start of the drying, ammonium sulphate and/or sulphuric acid is added to the fermentation broth in order to establish a sulphate/L-amino acid ratio of 0.85 to 1.2.

5. Method according to any of the preceding claims, **characterized in that** "partially or completely removing the biomass" is understood to mean removing at least 30% by weight, preferably at least 50% by weight, particularly at least 70% by weight, particularly preferably at least 90% by weight, of the biomass, wherein the biomass is preferably removed by ultrafiltration.

6. Method according to any of the preceding claims, **characterized in that** a biomass content is established of at most 4% by weight, preferably at most 3% by weight, particularly preferably at most 2% by weight, particularly at most 1% by weight.

7. Method according to any of the preceding claims, **characterized in that** the surface-active substance is present in the fermentation broth before drying in an amount of 0.025 to 20% by weight, particularly 0.1 to 20% by weight, preferably 0.3 to 10% by weight.

8. Method according to any of the preceding claims, **characterized in that** the water content in the fermentation broth is adjusted to a value of 35 to 50% by weight before drying, wherein the adjustment of the water content is preferably carried out by evaporation, reverse osmosis or nanofiltration.

9. Method according to any of the preceding claims, **characterized in that** the fermentation broth used in the drying has the following properties:
a) Biomasse content of at most 4% by weight, preferably at most 3% by weight, particularly preferably at most 2% by weight, especially preferably at most 1% by weight;
b) L-amino acid content, preferably L-lysine, (as amino acid base) of 12 to 48% by weight;
c) solids content (including biomass) of 20 to 60% by weight, preferably 30 to 50% by weight;
d) surface-active substance content of 0.025 to 20% by weight, preferably 0.1 to 20% by weight, particularly 0.3 to 10% by weight;
e) % by weight ratio of sulphate to L-amino acid, particularly L-lysine, of 0.8 to 1.2;
f) pH of 3.5 to 7.0, preferably 4.0 to 5.0.

10. Method according to any of the preceding claims, **characterized in that** the drying is conducted by spray-drying, preferably spray granulation, wherein a fluidized bed reactor is preferably used for the spray granulation.

11. Granular feed additive, comprising the following features:
a) L-amino acid content, preferably L-lysine, of at least 20% by weight, preferably 25 to 60% by weight, particularly 30 to 60 or 40 to 60% by weight, particularly preferably 45 to 55% by weight,
b) mean particle diameter of 60 to 2500 µm, preferably 60 to 1500 µm;
c) biomass content of at most 8% by weight, preferably at most 6% or 4% by weight, particularly preferably at most 3%, 2% or 1% by weight;
d) surface-active substance content of 0.15 to 35% by weight, preferably 0.15 to 30% by weight, particularly preferably 0.5 to 15% by weight comprising 3 to 25% by weight corn steep liquor or 0.15 to 10% by weight phospholids, polyglycols or mixtures thereof, wherein the corn steep liquor has a dry mass of at least 40% by weight and a residual sugar content of at most 2% by weight;
e) preferably a water content (residual moisture) of at most 3.5% by weight,
f) preferably a layer of edible oil coating the particle.

12. Feed additive according to Claim 11, **characterized in that** the L-amino acid is L-lysine, which is present at least in part as a sulphate salt and the molar ratio of sulphate to L-lysine is at least 0.5, preferably 0.8 to 1.2.

13. Feed additive according to any of the preceding claims, **characterized in that** said additive has less than 30% by volume, preferably less than 20% by volume, particularly preferably less than 15% by volume of cavities.

## Revendications

1. Procédé de fabrication d'un additif d'aliments pour animaux, **caractérisé en ce qu'**un bouillon de fermentation contenant un acide L-aminé, qui présente une teneur en eau de 35 à 75 % en poids et une teneur en substance tensioactive de 0,1 à 20 % en poids, et duquel la biomasse a été éliminée en partie ou en totalité, est transformé en une composition particulaire par séchage, au moins une des substances tensioactives suivantes étant contenue en la quantité suivante : de l'eau de trempe du maïs en une quantité de 1 à 10 % en poids, l'eau de trempe du maïs présentant une masse sèche d'au moins 40 % en poids et une teneur résiduelle en sucres d'au plus 2 % en poids ; des phospholipides, des polyglycols, ainsi que des mélanges de ceux-ci, chacun en une quantité de 0,1 à 5 % en poids.

2. Procédé de fabrication d'un additif d'aliments pour animaux contenant un acide L-aminé, comprenant les étapes suivantes :
a) la préparation d'un bouillon de fermentation contenant un acide L-aminé ;
b) l'élimination partielle ou totale de la biomasse du bouillon de fermentation ;
c) l'ajout d'une substance tensioactive au bouillon de fermentation après la fin de la fermentation et avant le début du séchage, de telle sorte que la substance tensioactive soit contenue avant le début du séchage dans le bouillon de fermentation en une quantité de 0,025 à 20 % en poids ;
d) le séchage du mélange obtenu en une composition particulaire ;
e) éventuellement le revêtement des particules avec une huile comestible, des particules qui sont revêtues en totalité ou en partie avec l'huile comestible étant obtenues.

3. Procédé selon la revendication 1 ou 2, **caractérisé en ce que** l'acide L-aminé consiste en la L-lysine, la L-méthionine, la L-thréonine, la L-valine ou le L-tryptophane, de préférence la L-lysine.

4. Procédé selon la revendication 1 ou 2, **caractérisé en ce que** l'acide aminé produit est la L-lysine et **en ce que** du sulfate d'ammonium et/ou de l'acide sulfurique sont ajoutés au bouillon de fermentation dans une étape de procédé après la fin de la fermentation et avant le début du séchage afin d'ajuster un rapport sulfate/acide L-aminé de 0,85 à 1,2.

5. Procédé selon l'une quelconque des revendications précédentes, **caractérisé en ce qu'**une « élimination partielle ou totale de la biomasse » doit être comprise comme une élimination d'au moins 30 % en poids, de préférence d'au moins 50 % en poids, notamment d'au moins 70 % en poids, de manière particulièrement préférée d'au moins 90 % en poids de la biomasse, l'élimination de la biomasse ayant de préférence lieu par ultrafiltration.

6. Procédé selon l'une quelconque des revendications précédentes, **caractérisé en ce qu'**une teneur en biomasse d'au plus 4 % en poids, de préférence d'au plus 3 % en poids, de manière particulièrement préférée d'au plus 2 % en poids, notamment d'au plus 1 % en poids, est ajustée.

7. Procédé selon l'une quelconque des revendications précédentes, **caractérisé en ce que** la substance tensioactive est contenue dans le bouillon de fermentation avant le séchage en une quantité de 0,025 à 20 % en poids, notamment de 0,1 à 20 % en poids, de préférence de 0,3 à 10 % en poids.

8. Procédé selon l'une quelconque des revendications précédentes, **caractérisé en ce que** la teneur en eau dans le bouillon de fermentation est ajustée à une valeur de 35 à 50 % en poids avant le séchage, l'ajustement de la teneur en eau ayant de préférence lieu par évaporation, osmose inverse ou nanofiltration.

9. Procédé selon l'une quelconque des revendications précédentes, **caractérisé en ce que** le bouillon de fermentation utilisé dans le séchage présente les propriétés suivantes :
a) une teneur en biomasse d'au plus 4 % en poids, de préférence d'au plus 3 % en poids, de manière particulièrement préférée d'au plus 2 % en poids, notamment d'au plus 1 % en poids ;
b) une teneur en acide L-aminé, de préférence en L-lysine, (en tant que base d'acide aminé) de 12 à 48 % en poids ;
c) une teneur en solides (y compris la biomasse) de 20 à 60 % en poids, de préférence de 30 à 50 % en poids ;
d) une teneur en substance tensioactive de 0,025 à 20 % en poids, de préférence de 0,1 à 20 % en poids, notamment de 0,3 à 10 % en poids ;
e) un rapport entre les % en poids de sulfate et d'acide L-aminé, notamment de L-lysine, de 0,8 à 1,2 ;
f) un pH de 3,5 à 7,0, de préférence de 4,0 à 5,0.

10. Procédé selon l'une quelconque des revendications précédentes, **caractérisé en ce que** le séchage a lieu par séchage par pulvérisation, de préférence par granulation par pulvérisation, un réacteur à lit fluidisé étant de préférence utilisé lors de la granulation par pulvérisation.

11. Additif granulaire d'aliments pour animaux, présentant les caractéristiques suivantes :
a) une teneur en acide L-aminé, de préférence en L-lysine, d'au moins 20 % en poids, de préférence de 25 à 60 % en poids, notamment de 30 à 60 ou de 40 à 60 % en poids, de manière particulièrement préférée de 45 à 55 % en poids,
b) un diamètre de grain moyen de 60 à 2 500 µm, de préférence de 60 à 1 500 µm ;
c) une teneur en biomasse d'au plus 8 % en poids, de préférence d'au plus 6 ou 4 % en poids, de manière particulièrement préférée d'au plus 3, 2 ou 1 % en poids ;
d) une teneur en substance tensioactive de 0,15 à 35 % en poids, de préférence de 0,15 à 30 % en poids, de manière particulièrement préférée de 0,5 à 15 % en poids, contenant 3 à 25 % en poids d'eau de trempe du maïs ou 0,15 à 10 % en poids de phospholipides, de polyglycols ou de mélanges de ceux-ci, l'eau de trempe du maïs présentant une masse sèche d'au moins 40 % en poids et une teneur résiduelle en sucres d'au plus 2 % en poids ;
e) de préférence une teneur en eau (humidité résiduelle) d'au plus 3,5 % en poids,
f) de préférence une couche d'huile comestible entourant le grain.

12. Additif d'aliments pour animaux selon la revendication 11, **caractérisé en ce que** l'acide L-aminé consiste en la L-lysine, celle-ci se présente au moins partiellement sous la forme d'un sel de sulfate, et le rapport molaire entre le sulfate et la L-lysine est d'au moins 0,5, de préférence de 0,8 à 1,2.

13. Additif d'aliments pour animaux selon l'une quelconque des revendications précédentes, **caractérisé en ce qu'**il comprend moins de 30 % en volume, de préférence moins de 20 % en volume, de manière particulièrement préférée moins de 15 % en volume, d'espaces vides.
